# EUROPEAN PATENT SPECIFICATION

(11) **EP 2 538 877 B1**
(45) Date of publication and mention of the grant of the patent: **07.10.2015**
(21) Application number: 11712008.9
(22) Date of filing: 18.02.2011
(51) Int. Cl.: A61F 2/18, A61F 5/08

(54) **NASAL DILATOR**
NASALER DILATATOR
DILATATEUR NASAL

(30) Priority: 24.02.2010 IT BO20100103
(43) Date of publication of application: 02.01.2013
(73) Proprietor: Mistro, Ilario, 40068 San Lazzaro Di Savena (Bologna) (IT)
(72) Inventor: TASCA, Ignazio, I-40024 Castel San Pietro Terme (bologna) (IT)
(74) Representative: Fanzini, Valeriano
(86) International application number: PCT/IB2011/050689
(87) International publication number: WO 2011/104660

(56) References cited:
- EP-A1- 0 958 798
- EP-A1- 1 105 047
- WO-A1-2006/047811
- WO-A1-2007/119041
- DE-U1- 29 718 838
- GB-A- 2 330 079
- NL-C2- 1 019 779
- US-A- 4 778 466

## Description

### Technical Field

This invention relates to a nasal dilator for increasing the patency of the nasal cavities and making it easier to breathe through the nose.

It is known that sleep apnoea and snoring are sometimes caused by a narrowing and at times occlusion of the nasal cavities.

### Background Art

To make breathing easier, above all while resting at night, there are prior art dilator devices which, when applied to the outside of the nose, help to widen the nasal cavities and allow increased air flow during breathing.

Such dilators - due to their physical structure and the fact that they are located outside the nose during use - can be positioned by the user without a precise and exactly repeatable reference.

Therefore, the resulting positioning has a certain degree of empiricism which does not allow the best use to be made of it relative to the precise anatomical shape and to the actual dimensions of the nose of each potential user. This condition is often also aggravated by a certain instability of the device which can easily be moved from the initial position assigned to it and which is, therefore, such that it reduces, or completely cancels out, the benefits to breathing deriving from its use.

There is also a prior art dilator which can be inserted in the nasal cavities through the user's nostrils to aid air flow, comprising a fork-shaped element with a transversal bridge designed to engage, during use, with the front face of the nasal septum. Extending longitudinally from the bridge there are arms which can be inserted in the corresponding nasal cavities and from whose free ends there extend laterally, cantilever-style, respective elastic elements, having a general arched shaped and designed to engage with and widen a corresponding zone of the nasal cavity. The elastic widening elements underpin a corresponding passage for air breathed.

However, this mechanical dilator, available only in one model, has arched elements which are too rigid and which do not adapt well to different types of nostrils, meaning that they may cause discomfort or pain for the user.

EP0958798 discloses a nasal dilator according to the preamble of claim 1.

### Disclosure of the Invention

It is proposed a new solution alternative to the prior art solutions known and, more specifically, a solution which can overcome one or more of the above-mentioned disadvantages or problems and/or satisfy one or more of the needs referred to above or which may be inferred from the above description.

Therefore, a mechanical dilator is provided, which can be inserted in the nasal cavities through the user's nostrils to aid air flow, comprising a fork-shaped element (3) having a bridge (5) designed, in practice, to engage with the front face of the nasal septum, arms (4) extending longitudinally from the bridge and being insertable in the nasal cavities, respective elastic elements (2) extending laterally cantilever-style from the free ends of the arms, said elastic elements having a general arched shape and being designed to engage with and widen a corresponding zone of the nasal cavity; the elastic widening elements (2) underpinning a passage for air breathed; the elastic elements (2) lying in two planes (6) which converge and are transversal to a third plane (7) in which the fork (3) lies; characterised in that the elastic widening elements (2) each comprise a first part (2a) which is substantially transversal, or horizontal, connected to the respective arm (4) of the fork and extending laterally from the arm, and an arched or generally arched portion (2b) for substantially engaging with the lateral wall of the nasal cavity and extending from the portion (2a) connected to the fork (3). In this way, it is possible to increase the flexibility of the elastic widening element and improve its adaptability to the particular nasal cavity

Other advantageous aspects of the invention are described in the other claims.

### Brief Description of the Drawings

The technical features of the invention are clearly described in the appended claims and its advantages are more apparent from the detailed description which follows, with reference to the accompanying drawings which illustrate several preferred, non-limiting example embodiments of the invention and in which:
- Figure 1A is a perspective view showing a dilator made according to the invention in an operating condition in the nose of the user;
- Figure 1B is an elevation view of the dilator made according to the invention;
- Figures 2 and 3 are respectively a front view and a rear view of the dilator;
- Figures 4 and 5A are respectively a top view and a bottom view of the dilator;
- Figure 5B is a cross-section according to the line V-V of Figure 5A;
- Figures 6 and 7 are three-dimensional illustrations of the dilator, seen from two different and opposite viewpoints;
- Figure 8 is a view of an alternative embodiment of the dilator, made in the form of a customisable gauging device;
- Figures 9 and 10 are a front view and a rear view of the dilator, customisable gauging device;
- Figures 11 and 12 are respectively a top view and a bottom view of the dilator of Figure 8;
- Figures 13 and 14 are three-dimensional illustrations of the dilator, customisable gauging device, seen from two different and opposite viewpoints;
- Figures 15 and 16 are an elevation view and a plan view of a container specifically designed to contain the dilators made according to the invention, the container being shown with some parts cut away to better illustrate others;
- Figures 17 and 18 are respectively a top plan view and an elevation view of a component part of the container of Figures 15 and 16;
- Figure 19 is a view of the container which is the opposite of that of Figures 17 and 18;
- Figure 20 is an elevation view of a lid for the container which holds the dilators;
- Figure 21 is an assembly view of the container in its entirety;
- Figures 22A, 22B, 22C are plan views of three different dilators forming a preferred embodiment of a set of dilators made according to this invention.

### Detailed Description of the Preferred Embodiments of the Invention

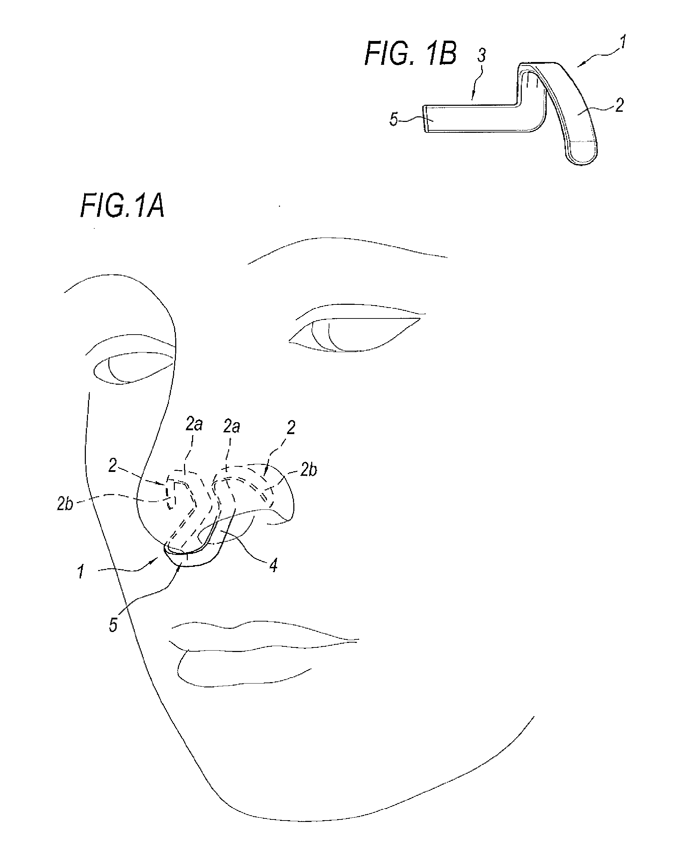

With reference to the accompanying drawings, in Figure 1, the numeral 1 denotes in its entirety a mechanical anatomical dilator, which can be inserted in the nasal cavities, through the nostrils, to widen their transit cross-section and aid air flow, consequently making it easier for the user wearing the dilator 1 to breathe through his nose.

Therefore, this mechanical dilator can be inserted in the nasal cavities through the user's nostrils to aid air flow, and comprises a fork-shaped element 3 with a transversal bridge 5 designed, during use, to engage with the front face of the nasal septum, and from which there extend longitudinally corresponding arms 4 which can be inserted in the corresponding nasal cavities, from the free ends of the arms there extending laterally, cantilever-style, or in opposite lateral directions, respective elastic elements 2, having a general arched shape and designed to engage with and widen a corresponding zone of the nasal cavity. The elastic widening elements 2 underpin a corresponding passage for air breathed.

Therefore, as illustrated, the dilator 1 - preferably made of plastic material - basically comprises two sprung elastic elements 2, which are supported by a fork 3 positioned between them.

As illustrated, in particular, the fork 3 has two straight arms 4, converging and connected to each other by an intermediate bridge 5.

At their ends furthest from the bridge 5 and close to each other, the arms 4 support the elastic elements 2, which extend cantilever-style, projecting from the two opposite sides of the fork 3, outwards from the arms 4 of the fork.

The elastic elements 2 have an elongate arched shape - roughly semicircular or parabolic - and extend in two planes 6, which converge and are transversal to a third plane 7, substantially at right angles to the first planes, in which the fork 3 assembly lies.

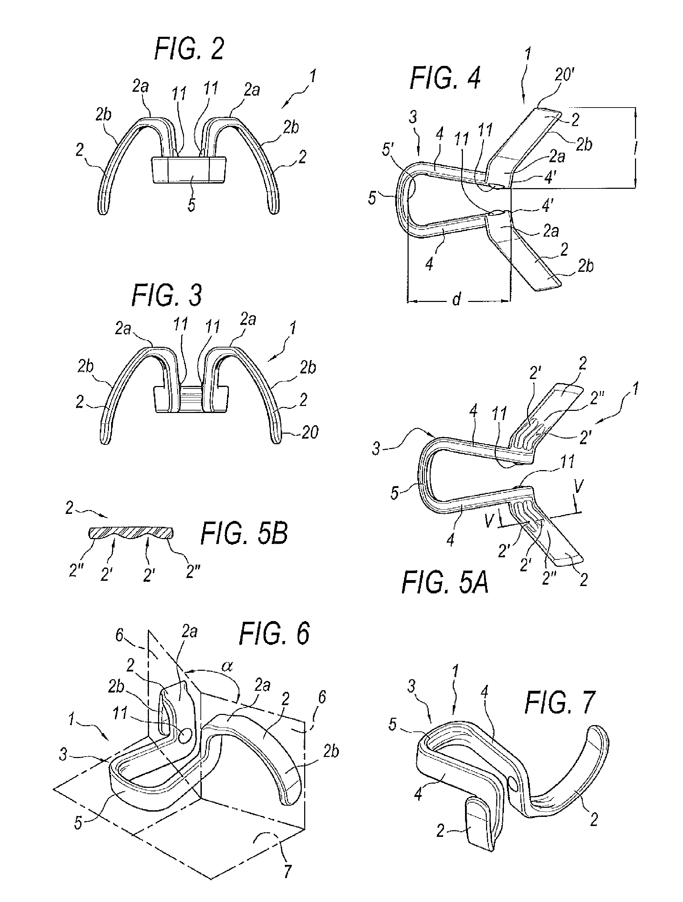

As Figures 2 and 3 clearly show, the elastic elements 2 have mixtilineal arched profiles, with variable curvatures along their extension.

The arched profiles are preferably curved and shaped to match the internal anatomical profile of the nasal cavities, detected using statistics, and by experimentation.

In more detail, the elastic widening elements 2 each comprise a first part 2a which is substantially transversal, or horizontal, connected to the respective arm 4 of the fork and extending laterally from the arm, and an arched or generally arched portion 2b for substantially engaging with the lateral wall of the nasal cavity and extending from the portion 2a connected to the fork 3.

Advantageously, as may be inferred with reference to Figures 5A and 5B, the elastic widening elements 2, 2 comprise a stretch with a weakened section.

In this way, it is possible to increase the flexibility of the elastic widening element and improve its adaptability to the particular nasal cavity of the user. Moreover, in this way, the dilator can be worn for lengthy periods without causing the user any damage or discomfort.

As illustrated, the stretch with a weakened section is formed by corresponding recesses 2' which extend from the lower face 2" of the respective stretch of the widening element into the body of the elastic tab 2.

In particular, as illustrated, the stretch with a weakened section, or the corresponding recesses 2', extend, or are provided, close to the point where the element 2 is connected to the respective arm 4.

Moreover, as illustrated, the stretch with a weakened section, or provided with recesses 2', extends on the portion 2a extending from the respective arm 4 of the fork 3.

Moreover, as illustrated, the stretch with a weakened section, or provided with recesses 2', extends, from the connecting portion 2a, for a predetermined length of the arched portion 2b which engages with the lateral wall of the nasal canal or cavity.

As is clear in Figure 1A, the dilator 1 is worn by inserting the two sprung elastic elements 2 in the nostrils, then gently pushing them up into the nasal cavities as far as the bridge allows 5.

Once the bridge makes contact with the bottom of the nose, it stops insertion of the dilator, resulting in definitive and stable positioning of the dilator 1, that is to say, of the elastic elements 2 in the nasal cavities, where the elastic reaction of the spring - tending to spontaneously straighten the arched profile - causes widening and stable dilation in that condition.

To prevent the dilator from spontaneously slipping out backwards, the end of the arms 4 which are closest to each other are provided with contact elements 11 substantially supported at the connecting zone between the arms 4 and the elastic elements 2. The elements 11 are such that they interact with the internal anatomical surface of the nose and prevent the dilator 1 from slipping backwards towards the bottom of the nose.

To take into account the various and most diverse anatomical shapes and dimensions of different users, the dilator 1 is made with variable dimensions and, more particularly, with arms 4 which have a length, measured between the connecting bridge 5 and the elastic elements 2, that may vary according to the anatomy of the user.

This adaptability and customisability of the dilator 1 to the shape of various noses also includes the idea of making the thickness of the elastic elements 2 variable, and in particular decreasing, from the zone where they are connected to the arms 4 to their free ends.

This gives the elastic elements 2 stiffness which can vary according to their longitudinal extension, so that it is possible to graduate the intensity of the elastic reaction developed by the elastic elements 2 from one end to the other of their structure.

In Figures 1B to 7, the dilator 1 in its entirety appears to have a single-piece structure: none of its component parts can be disconnected from the others. The purpose of this is to guarantee maximum safety during use, without the risk that the dilator 1 or its parts can be inhaled by the user.

Therefore, the dilator preferably and advantageously has a fork 3 comprising, between the inner or rear face 5' of the bridge portion and the rear end 4' of the fork 3, or of its arms 4, a length "d" which is suitably selected and is suited to the particular shape of the nose of a respective class or group of users.

In practice, by providing a nasal dilator with a specific and predetermined length "d", it is possible to guarantee a respective class or group of users, having similarly shaped nasal cavities, excellent air flow.

Moreover, according to a preferred embodiment, the dilator, advantageously and preferably, comprises a widening element 2 which, between the inner face 11 of the fork 3 at the end connected to the widening element 2 and the outer end 20' of the widening element 2, has a width "1" which is suitably selected and is suited to the particular shape of the nose of a respective class or group of users.

In practice, by providing a nasal dilator with a specific and predetermined width "1", it is possible to guarantee a respective class of users, having similarly shaped nasal cavities, optimum nasal dilator lodging in the nasal cavities.

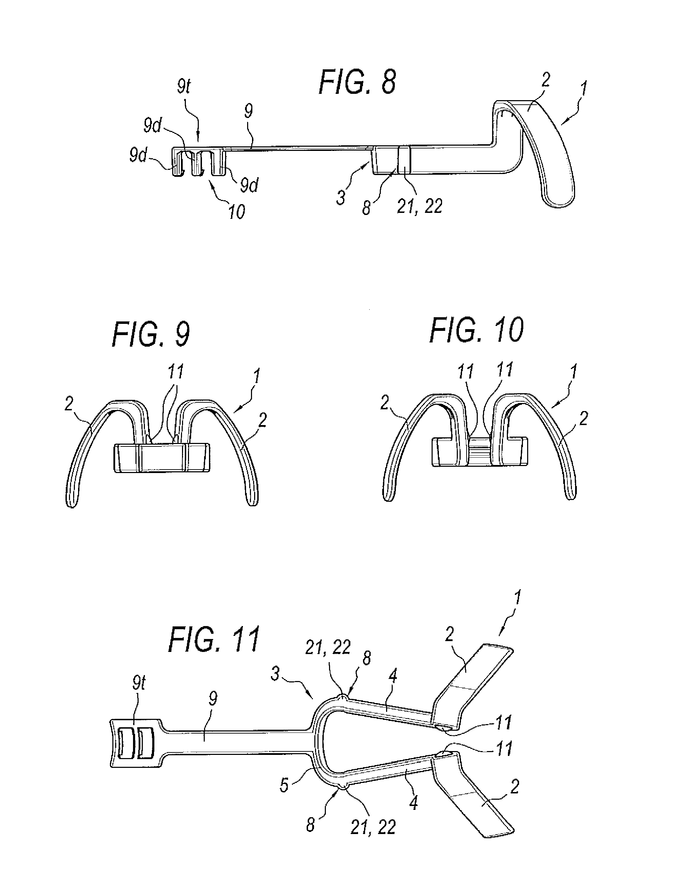

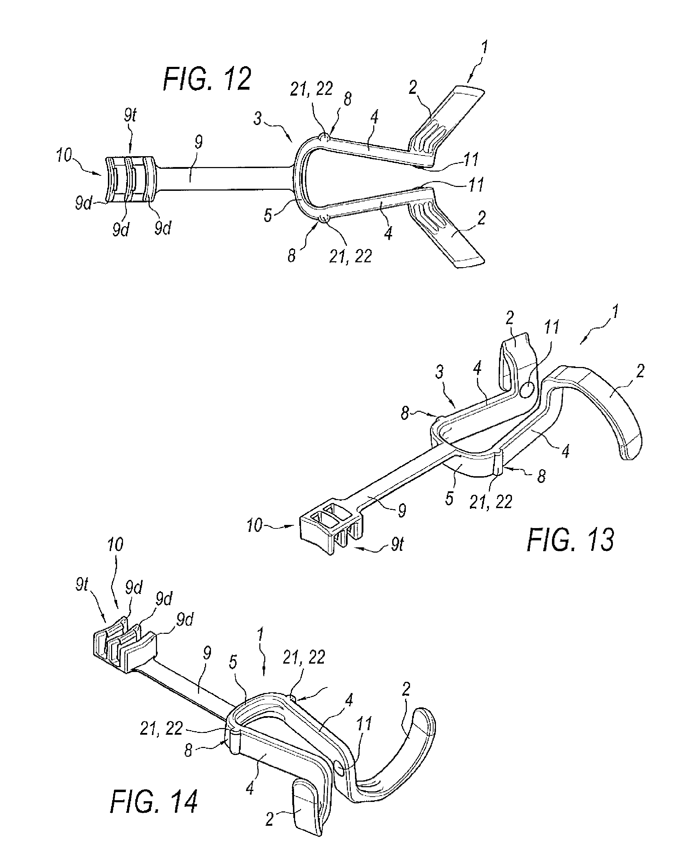

Figures 8 to 13 show a different type of dilator 1, substantially similar to the dilator of the version previously illustrated in Figures 1B to 7, but in which, unlike what was previously shown, the bridge 5 and the arms 4 are connected by removable connections 8, for example shaped couplings, comprising projections 21 and cavities 22 which match each other geometrically and can penetrate each other.

Such a type of dilator 1 is particularly useful to the specialist doctor for gauging the specific dilator 1 to be prescribed for the patient relative to his morphology, as well as his personal respiratory problems.

By alternately fitting bridges 5 having different thickness and a different shape, using the bottom of the nose as a positioning reference, it is possible to position the elastic elements in the nasal labyrinths furthest from or closest to the bottom of the nose, according to requirements.

Obviously, if required, it is also possible to fit on the bridge 5 arms 4 converging with different angles, and arms 4 having elastic elements 2 with various profiles, stiffness and lengths.

To promote the interchangeability of the bridges 5, the dilator 1 gauging device, described herein, is provided with an easy grip element 9 which is also used for manually holding the bridge 5.

The grip element 9 preferably has an elongate rectangular shape, with two end heads 9t.

At the heads 9t, the grip and holding element 9 comprises elastic snap-on joints 10, comprising small teeth 9d projecting from the heads 9t, which allow the grip element to be removably connected to the bridge 5 or to the various types of bridge 5 to be tried.

Obviously, the grip element 9 is also a valid aid for the doctor for inserting the dilator 1 gauging device in the nose of the patient.

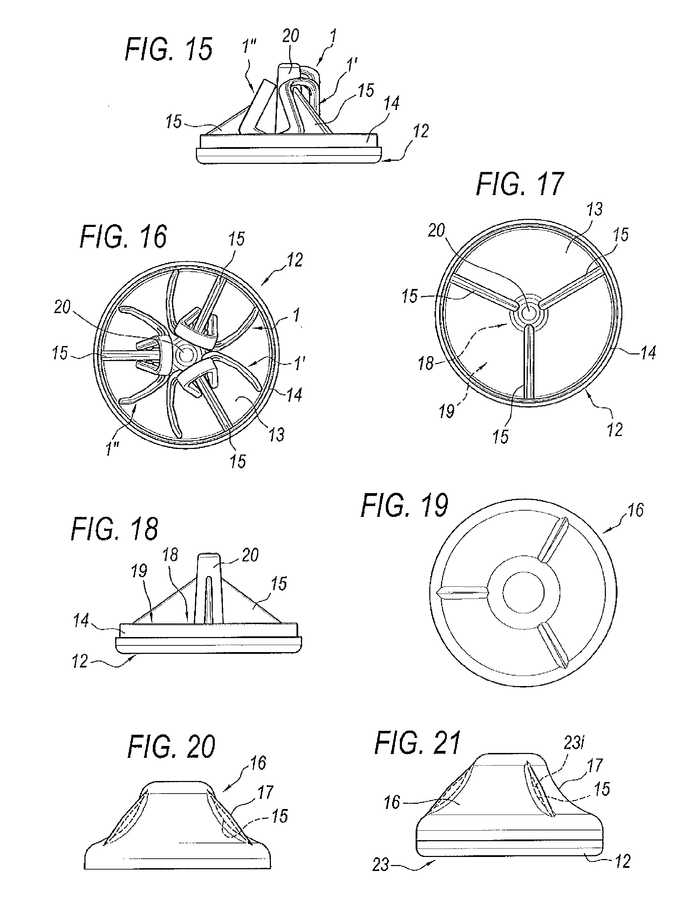

A container 23 is also provided, shown in Figures 15 to 21, basically comprising a box-shaped base 12, having a circular bottom surface 13 and an edge 14 which at least partly borders on the bottom surface 13 and which rises from the latter, surrounding it completely. The box-shaped base 12 is provided with a set of dividers 15, raised above the bottom surface 13, which are distributed radially to the base 12 and evenly along its circumference.

The dividers 15 are positioned at a central partition 19 of the box-shaped base 2 from which a central element 20 rises, projecting from the bottom surface 13 and which together with the surrounding edge 14 helps to make the dividers 15 integral with the box-shaped body 12.

As shown in Figure 18, the dividers 15 preferably have the structure of flat elements with a triangular outline, sloping downwards from the central partition 20 towards the edge 14 surrounding the base 12.

Inside the box-shaped base 12, the dilators 1 are stably but removably accommodated with the elastic elements 2 resting on the bottom surface 13 and simultaneously in contact with the lateral edge 14, and finally the fork 3 is elastically clamped on the divider 15 which is raised above the bottom surface 13.

The container 23 also comprises a lid 16, which can be connected to the box-shaped base 12 by concentric contact with the edge 14.

Moreover, a comparison of Figures 15, 20 and 21 reveals that the lid 16 of the container 23 comprises a bell-shaped lateral surface 17, positioned opposite the dividers 15 and sloping downwards parallel with them, but without making contact with them.

In that way, gaps 23 are created between the surface 17 and the dividers 15. The bridge 5 of a dilator 1 is interposed in each gap. When the lid 16 is connected to the box-shaped base 12 to close the container 23, the bridge 5 remains locked in position, thus immobilising the dilator 1 which is stably held in the position assigned to it.

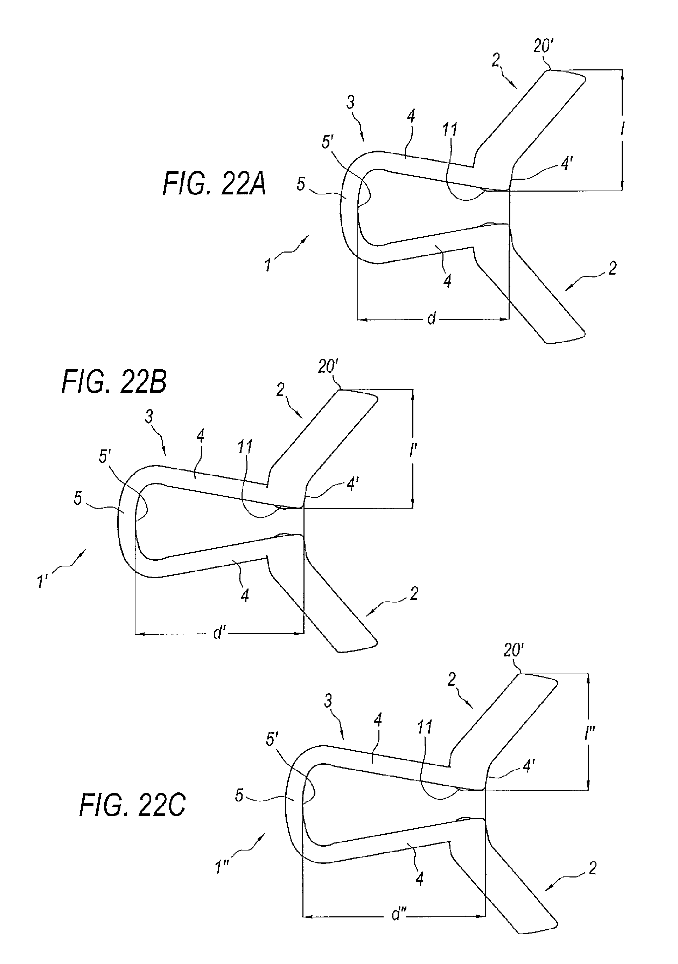

As may be inferred from Figures 22A to 22C, the container supports an advantageous set of nasal dilators, in which the dilators are shaped as shown in Figures 1B to 7.

Therefore, the set of dilators comprises a first and a second nasal dilator 1, 1', having respective longitudinal lengths d, d' that are different and which can be used by users, or classes or groups of users, having a different nasal shape.

In practice, each dilator 1 and 1' has a respective fork 3 comprising, between the inner, or rear, face 5' of the bridge portion and the respective rear end 4' of the fork 3, or of its arms 4, a corresponding length d or d', the lengths d or d' of the dilators being different to each other and suitably selected or suited to the particular shape of the nose of respective classes or groups of users, thus guaranteeing each respective group of users excellent air flow.

Moreover, in this set, the first and second nasal dilators 1, 1' have respective widths 1, 1' of the elastic widening elements 2 for the nasal cavity which are different and can be used by classes or groups of users having a respective and different nasal shape.

In practice, each dilator 1 and 1' comprises a respective widening element 2, which, between the inner face 11 of the fork 3 at the end connected to the widening elements 2 and the outer end 20' of the widening element 2, has a corresponding width 1 and 1', the widths 1 or 1' of the dilators being different and suitably selected, or suited to the particular shape of the nose of respective classes or groups of users, thus guaranteeing each group of users optimum dilator lodging in the respective nasal cavity.

In the embodiment illustrated, the set of nasal dilators also comprises a third nasal dilator 1" in turn having a respective longitudinal length d", between the respective inner, or rear, face 5' of the bridge portion and the respective rear end 4' of the fork 3, or of its arms 4, whose length d" is different to the corresponding lengths d and d' of the first and second dilators 1, 1' and which can be used by a group of users having yet another different nasal shape, thus guaranteeing a further group of users excellent air flow.

This third nasal dilator 1" also has a respective width 1", between the inner face 11 of the fork 3 at the end connected to the widening elements 2 and the outer end 20' of the widening element 2, said width 1" being different to the widths 1 and 1' of the first and second dilators, and which can be used by a group of users having yet another different nasal shape, thus guaranteeing a further group of users optimum dilator lodging in the respective nasal cavity.

Therefore, advantageously, each respective dilator 1, 1', 1" in the set of nasal dilators may have a longitudinal length d, d', d" and a width 1, 1', 1" of the arched portion which are suitably predetermined or suited to the particular shape of the nose of respective classes or groups of users.

In this way, without going to a specialist doctor, an individual user may, by trying the dilators 1, 1' and 1" in this set, identify amongst them the dilator which is best suited to the shape of his nose, thus eliminating the risk of using an ineffective dilator device.

Obviously, this set of dilators could comprise a number of dilators greater than the three illustrated herein, each having a respective length and width.

This nasal dilator is made of plastic material, preferably acetal resin, although it could be made of any other material, for example, even carbon fibre.

This dilator can be positioned by the user with absolute precision and repeatability.

This dilator can be inserted in the nasal cavities and positioned with precise reference to the end of the user's nose. All in a completely safe way which prevents accidental inhalation of the dilator and/or its component parts.

The material for making this nasal dilator is preferably biocompatible, or in any case, non-toxic for the body. In particular, a food-safe material could be used.

However, this nasal dilator is preferably made of polypropylene with the addition of a thermoplastic elastomer.

According to another embodiment, a shaped supporting core could be made which is covered with a soft or elastic layer, for example made of silicone, the latter therefore reproducing the above-mentioned shapes of this dilator. The supporting core could be made of carbon fibre, in the form of a titanium shaped plate, or in the form of a stainless steel shaped plate.

According to another embodiment, the material used to make this nasal dilator could be, entirely or partly, a biodegradable material.

The invention described is susceptible of industrial application. It would be obvious to one skilled in the art that several changes and/or modifications can be made to the invention without departing from the scope of the invention, described in depth above. In particular, one skilled in the art could easily imagine further embodiments of the invention comprising one or more of the features described herein. It will also be understood that all the details of the invention may be substituted by technically equivalent elements.

## Claims

1. A mechanical dilator, which can be inserted in the nasal cavities through the user's nostrils to aid air flow, comprising a fork-shaped element (3) having a bridge (5) designed, in practice, to engage with the front face of the nasal septum, arms (4) extending longitudinally from the bridge and being insertable in the nasal cavities, respective elastic elements (2) extending laterally cantilever-style from the free ends of the arms, said elastic elements having a general arched shape and being designed to engage with and widen a corresponding zone of the nasal cavity; the elastic widening elements (2) underpinning a passage for air breathed; the elastic elements (2) lying in two planes (6) which converge and are transversal to a third plane (7) in which the fork (3) lies; **characterised in that** the elastic widening elements (2) each comprise a first part (2a) which is substantially transversal, or horizontal, connected to the respective arm (4) of the fork and extending laterally from the arm, and an arched or generally arched portion (2b) for substantially engaging with the lateral wall of the nasal cavity and extending from the portion (2a) connected to the fork (3).

2. The dilator according to claim 1, **characterised in that** the elastic widening elements (2) comprise a stretch having a weakened section.

3. The dilator according to claim 2, **characterised in that** the stretch with a weakened section is close to the point of connection to the respective arm.

4. The dilator according to either of the foregoing claims 2 and 3, **characterised in that** the weakened section is formed by recesses extending from the lower face of the respective stretch of the widening element (2).

5. The dilator according to any of the foregoing claims from 2 to 4, **characterised in that** the respective widening element (2) comprises said substantially transversal portion (2a) connected to the respective arm (4) of the fork.

6. The dilator according to claim 5, **characterised in that** the stretch with a weakened section extends on the portion (2a) of the widening element (2) extending from the respective arm (4) of the fork (3).

7. The dilator according to claim 5 or 6, **characterised in that** the stretch with a weakened section extends on the portion (2b) of the widening element (2) which engages with the lateral part of the nasal cavity.

8. The dilator according to any of the foregoing claims, **characterised in that** the dilator (1) fork comprises, between the inner or rear face (5') of the bridge portion and the rear end (4') of the fork, a longitudinal length (d) which is suitably selected and is suited to the particular shape of the nose of a respective class or group of users; and/or being **characterised in that** the widening element (2) comprises, between the inner face (11) of the fork (3) at the end connected to the widening element (2) and the outer end (20') of the widening element (2), a width (1) which is suitably selected and is suited to the particular shape of the nose of a respective class or group of users.

9. The dilator according to any of the foregoing claims, **characterised in that** the arched shapes of the elastic elements (2) have mixtilineal profiles, with variable curvature.

10. The dilator according to any of the foregoing claims, **characterised in that** the elastic elements (2) have variable stiffness, according to their longitudinal extension, from the zone connected to the arms (4) to the free end.

11. The dilator according to any of the foregoing claims, **characterised in that** the bridge (5), the arms (4) and the elastic elements (2) together form a single piece.

12. The dilator according to any of the foregoing claims, **characterised in that** it comprises an easy grip element (9) which is also used for manually holding the bridge (5).

13. The dilator according to claim 12, **characterised in that** it comprises at least one joint (10) having an elastic snap-on action for removably connecting the grip element and the bridge (5).

14. The dilator according to any of the foregoing claims, **characterised in that** it comprises elements (11) designed to make contact with the anatomical surface inside the nostril, said elements being supported by the arms (4), substantially at the connecting zone between the arms (4) and the elastic elements (2).

15. The dilator according to any of the foregoing claims, **characterised in that** the thickness of the elastic elements (2) is variable.

## Patentansprüche

1. Mechanischer Dilatator, der durch die Nasenlöcher des Nutzers in die Nasenhöhlen eingesetzt werden kann, um die Luftströmung zu unterstützen, umfassend ein gabelförmiges Element (3), aufweisend eine Brücke (5), ausgestaltet, um in der Praxis mit der Frontseite der Nasenscheidewand in Eingriff zu gelangen, Arme (4), sich erstreckend längs von der Brücke und einfügbar in die Nasenhöhlen, jeweilige elastische Elemente (2), sich erstreckend seitlich hebelartig von den freien Enden der Arme, wobei die elastischen Elemente eine allgemeine Bogenform aufweisen und ausgestaltet sind, um mit der entsprechenden Zone der Nasenhöhle in Eingriff zu gelangen und diese zu erweitern;
wobei die elastischen Erweiterungselemente (2) einen Durchgang für die eingeatmete Luft stützen und die elastischen Elemente (2) in zwei Ebenen (6) liegen, die konvergierend und quer zu einer dritten Ebene (7) angeordnet sind, in der die Gabel (3) liegt, **dadurch gekennzeichnet, dass** die elastischen Erweiterungselemente (2) jeweils einen ersten Teil (2a) umfassen, der im Wesentlichen quer oder waagerecht verläuft und mit dem jeweiligen Arm (4) der Gabel verbunden ist und sich seitlich vom Arm erstreckt, sowie einen gebogenen oder allgemein gebogenen Abschnitt (2b) um im Wesentlichen mit der Seitenwind der Nasenhöhle in Eingriff zu gelangen und sich erstreckend vom mit der Gabel (3) verbundenen Abschnitt.

2. Dilatator nach Anspruch 1, **dadurch gekennzeichnet, dass** die elastischen Erweiterungselemente (2) ein Teilstück umfassen, dass eine verdünnte Sektion aufweist.

3. Dilatator nach Anspruch 2, **dadurch gekennzeichnet, dass** das Teilstück mit der verdünnten Sektion nah am Verbindungspunkt mit dem jeweiligen Arm angeordnet ist.

4. Dilatator nach einem der vorhergehenden Ansprüche 2 und 3, **dadurch gekennzeichnet, dass** die verdünnte Sektion durch Einschnitte gebildet ist, die sich von der unteren Seite des jeweiligen Teilstücks des Erweiterungselements (2) erstrecken.

5. Dilatator nach einem der vorhergehenden Ansprüche 2 bis 4, **dadurch gekennzeichnet, dass** das jeweilige Erweiterungselement (2) den im Wesentlichen quer verlaufenden Abschnitt (2a) umfasst, der mit dem jeweiligen Arm (4) der Gabel verbunden ist.

6. Dilatator nach Anspruch 5, **dadurch gekennzeichnet, dass** sich das Teilstück mit der verdünnten Sektion auf dem Abschnitt (2a) des Erweiterungselements (2) erstreckt, das sich vom jeweiligen Arm (4) der Gabel (3) erstreckt.

7. Dilatator nach Anspruch 5 oder 6, **dadurch gekennzeichnet, dass** sich das Teilstück mit der verdünnten Sektion auf dem Abschnitt (2b) des Erweiterungselements (2) erstreckt, mit dem seitlichen Teil der Nasenhöhle im Eingriff ist.

8. Dilatator nach einem der vorhergehenden Ansprüche, **dadurch gekennzeichnet, dass** die Gabel des Dilatators (1) zwischen der inneren oder rückseitigen Fläche (5') des Brückenabschnitts und dem rückseitigen Ende (4') der Gabel eine längsseitige Länge (d) umfasst, die geeignet ausgewählt ist und passend auf die besondere Form der Nase einer jeweiligen Klasse oder Gruppe von Nutzern abgestimmt ist, und/oder **dadurch gekennzeichnet, dass** das Erweiterungselement (2) zwischen der inneren Fläche (11) der Gabel (3) an dem mit dem Erweiterungselement (2) verbundenen Ende und dem äußeren Ende (20') des Erweiterungselements (2) eine Breite (1) umfasst, die geeignet ausgewählt ist und passend auf die besondere Form der Nase einer jeweiligen Klasse oder Gruppe von Nutzern abgestimmt ist.

9. Dilatator nach einem der vorhergehenden Ansprüche, **dadurch gekennzeichnet, dass** die gebogenen Formen der elastischen Elemente (2) gemischtlinige Profile mit einer variablen Krümmung aufweisen.

10. Dilatator nach einem der vorhergehenden Ansprüche, **dadurch gekennzeichnet, dass** die elastischen Elemente (2) eine variable Steifigkeit aufweisen, je nach ihrer Längsausdehnung, von der Zone, verbunden mit den Armen (4), zum freien Ende.

11. Dilatator nach einem der vorhergehenden Ansprüche, **dadurch gekennzeichnet, dass** die Brücke (5), die Arme (4) und die elastischen Elemente (2) zusammen ein einziges Stück formen.

12. Dilatator nach einem der vorhergehenden Ansprüche, **dadurch gekennzeichnet, dass** er ein einfaches Greifelement (9) umfasst, das auch genutzt wird, um die Brücke (5) mit der Hand zu halten.

13. Dilatator nach Anspruch 12, **dadurch gekennzeichnet, dass** er mindestens ein Verbindungsstück (10) umfasst, aufweisend eine elastische Schnappwirkung, um das Greifelement und die Brücke (5) entfernbar zu verbinden,

14. Dilatator nach einem der vorhergehenden Ansprüche, **dadurch gekennzeichnet, dass** er Elemente (11) umfasst, die ausgestaltet sind, um einen Kontakt zur anatomischen Oberfläche im Nasenloch herzustellen,
wobei diese Elemente von den Armen (4) gestützt werden, im Wesentlichen an der Verbindungszone zwischen den Armen (4) und den elastischen Elementen (2).

15. Dilatator nach einem der vorhergehenden Ansprüche, **dadurch gekennzeichnet, dass** die Dicke der elastischen Elemente (2) variabel ist.

## Revendications

1. Dilatateur mécanique pouvant être inséré dans les cavités nasales par les narines de l'utilisateur pour optimiser le passage de l'air, comprenant un élément fourchu (3) avec un pont (5) conçu, en pratique, pour s'engager avec la partie antérieure de la cloison nasale, des branches (4) se développant longitudinalement depuis le pont et pouvant être insérées dans les cavités nasales, des éléments élastiques respectifs (2) se développant latéralement en. porte-à-faux depuis les extrémités libres des branches, lesdits éléments élastiques ayant une forme générale cambrée et étant conçus pour s'engager avec et pour élargir une zone correspondante de la cavité nasale;
les éléments élastiques d'élargissement (2) soutenant un passage pour l'air respiré; les éléments élastiques (2) se situant sur deux plans (6) convergents et transversaux à un troisième plan (7) sur lequel se trouve l'élément fourchu (3); **caractérisé en ce que** les éléments élastiques d'élargissement (2) comportent chacun une première partie (2a) qui est essentiellement transversale, ou horizontale, raccordée à la branche respective (4) de l'élément fourchu et se développant latéralement depuis la branche, et une portion cambrée ou généralement cambrée (2b) pour s'engager essentiellement avec la paroi latérale de la cavité nasale et se développant depuis la portion (2a) raccordée à l'élément fourchu (3),

2. Dilatateur selon la revendication 1, **caractérisé en ce que** les éléments élastiques d'élargissement (2) comprennent une bande avec une section fragilisée.

3. Dilatateur selon la revendication 2, **caractérisé en ce que** la bande avec une section fragilisée est proche du point de raccordement à la branche respective.

4. Dilatateur selon l'une quelconque des revendications précédentes 2 et 3, **caractérisé en ce que** la section fragilisée consiste en des renfoncements se développant depuis la partie inférieure de la bande respective de l'élément d'élargissement (2).

5. Dilatateur selon l'une quelconque des revendications précédentes de 2 à 4, **caractérisé en ce que** l'élément d'élargissement respectif (2) comprend ladite portion essentiellement transversale (2a) raccordée à la branche respective (4) de l'élément fourchu.

6. Dilatateur selon la revendication 5, **caractérisé en ce que** la bande avec une section fragilisée se développe sur la portion (2a) de l'élément d'élargissement (2) se développant depuis la branche respective (4) de l'élément fourchu (3).

7. Dilatateur selon les revendications 5 ou 6, **caractérisé en ce que** la bande avec une section fragilisée se développe sur la portion (2b) de l'élément d'élargissement (2) qui s'engage avec la partie latérale de la cavité nasale.

8. Dilatateur selon l'une quelconque des revendications précédentes, **caractérisé en ce que** l'élément fourchu du dilatateur (1) comprend, entre la partie interne ou postérieure (5') de la portion du pont et l'extrémité postérieure (4') de l'élément fourchu, une longueur longitudinale (d) qui est correctement sélectionnée et convient à la forme particulière du nez d'une classe ou d'un groupe respectifs d'utilisateurs; et/ou **caractérisé en ce que** l'élément d'élargissement (2) comprend, entre la partie interne (11) de l'élément fourchu (3) sur l'extrémité raccordée à l'élément d'élargissement (2) et l'extrémité externe (20') de l'élément d'élargissement (2), une largeur (1) qui est correctement sélectionnée et convient à la forme particulière du nez d'une classe ou d'un groupe respectifs d'utilisateurs.

9. Dilatateur selon l'une quelconque des revendications précédentes, **caractérisé en ce que** les formes cambrées des éléments élastiques (2) ont des profils mixtilignes, avec courbure variable.

10. Dilatateur selon l'une quelconque des revendications précédentes, **caractérisé en ce que** les éléments élastiques (2) ont une rigidité variable selon leur développement longitudinal, depuis la zone raccordée aux branches (4) jusqu'à l'extrémité libre.

11. Dilatateur selon l'une quelconque des revendications précédentes, **caractérisé en ce que** le pont (5), les branches (4) et les éléments élastiques (2) forment ensemble une seule pièce.

12. Dilatateur selon l'une quelconque des revendications précédentes, **caractérisé en ce qu'**il comprend un élément de prise facile (9) qui sert également à tenir le pont (5) dans la main.

13. Dilatateur selon la revendication 12, **caractérisé en ce qu'**il comprend au moins un raccord (10) exerçant une action d'encliquetage élastique pour raccorder, de façon amovible, l'élément de prise et le pont (5).

14. Dilatateur selon l'une quelconque des revendications précédentes, **caractérisé en ce qu'**il comprend des éléments (11) conçus pour faire contact avec la surface anatomique à l'intérieur de la narine, lesdits éléments étant soutenus par les branches (4), essentiellement au niveau de la zone de raccordement entre les branches (4) et les éléments élastiques (2).

15. Dilatateur selon l'une quelconque des revendications précédentes, **caractérisé en ce que** l'épaisseur des éléments élastiques (2) est variable,
